(19) Europäisches Patentamt
European Patent Office
Office européen des brevets

(11) Veröffentlichungsnummer : **0 023 651**
**B1**

(12) # EUROPÄISCHE PATENTSCHRIFT

(45) Veröffentlichungstag der Patentschrift : **26.01.83**

(51) Int. Cl.³ : **C 07 D233/60**, A 01 N 43/50

(21) Anmeldenummer : **80104238.3**

(22) Anmeldetag : **18.07.80**

(54) **Imidazolyl-enolether, Verfahren zu ihrer Herstellung und ihre Verwendung als Fungizide.**

(30) Priorität : **04.08.79 DE 2931665**

(43) Veröffentlichungstag der Anmeldung : **11.02.81 Patentblatt 81/06**

(45) Bekanntmachung des Hinweises auf die Patenterteilung : **26.01.83 Patentblatt 83/04**

(84) Benannte Vertragsstaaten : **AT BE CH DE FR GB IT LI NL SE**

(56) Entgegenhaltungen : **DE A 2 720 949**

(73) Patentinhaber : **BAYER AG**
**Zentralbereich Patente, Marken und Lizenzen**
**D-5090 Leverkusen 1, Bayerwerk (DE)**

(72) Erfinder : **Draber, Wilfried, Dr.**
**In den Birken 81**
**D-5600 Wuppertal 1 (DE)**
Erfinder : **Büchel, Karl Heinz, Prof., Dr.**
**Haus an der Dachsdelle Dabringhauserstrasse 42**
**D-5093 Burscheid (DE)**
Erfinder : **Frohberger, Paul-Ernst, Dr.**
**Willi-Baumeister-Strasse 5**
**D-5090 Leverkusen (DE)**
Erfinder : **Brandes, Wilhelm, Dr.**
**Eichendorffstrasse 3**
**D-5623 Leichlingen (DE)**

**EP 0 023 651 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imidazolyl-enolether, Verfahren zu ihrer Herstellung und ihre Verwendung als Fungizide

Die Erfindung betrifft neue Imidazolyl-enolether, ein Verfahren zu ihrer Herstellung und ihre Verwendung als Fungizide.

Es ist bereits bekannt geworden, daß Imidazolyl-ether, wie beispielsweise substituierte 3,3-Dimethyl-1-(imidazol-1-yl)-1-phenoxy-2-(R-oxy)-butane, gute fungizide Eigenschaften aufweisen (vergleiche DE-OS 27 20 949). Die Wirkung dieser Verbindungen ist jedoch, insbesondere bei niedrigen Aufwandmengen und -konzentrationen, nicht immer ganz befriedigend.

Es wurden neue Imidazolyl-enolether der allgemeinen Formel I

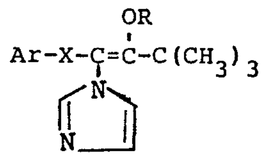

(I)

in welcher

Ar für Phenyl und Naphthyl steht, wobei diese Reste substituiert sein können durch Halogen, Alkyl mit 1 bis 6 und Alkoxy mit 1 bis 4 Kohlenstoffatomen, Halogenalkyl mit 1 bis 4 Kohlenstoff- und 1 bis 5 Halogenatomen, ferner durch Nitro, Cyano und schließlich durch gegebenenfalls halogensubstituiertes Phenyl,

R für Alkyl mit 1 bis 4 Kohlenstoffatomen steht und

X für Sauerstoff oder für die Methylengruppe steht, sowie deren Säure- und Metallsalz-Additionsverbindungen gefunden.

Die erfindungsgemäßen Verbindungen der allgemeinen Formel I kommen in den geometrischen Isomeren E(trans) und Z(cis) vor. Bei der E,Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, ist die Konfiguration Z (abgeleitet von zusammen), stehen sie auf entgegengesetzter Seite, E(abgeleitet von entgegen). Sowohl die einzelnen Isomeren als auch die Gemische werden erfindungsgemäß beansprucht.

Weiterhin wurde gefunden, daß man die Imidazolylenolether der allgemeinen Formel I erhält, wenn man Imidazolyl-ketone der allgemeinen Formel II

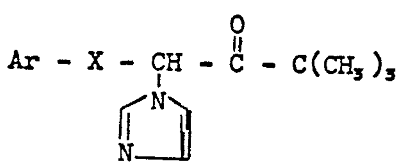

(II)

in welcher

Ar und X die oben angegebene Bedeutung haben, mit entsprechenden Alkylsulfaten bzw. -halogeniden in Gegenwart einer Base und in Gegenwart eines organischen Verdünnungsmittels, oder in einem wässrig-organischen Zweiphasensystem in Gegenwart eines Phasentransferkatalysators umsetzt, und gegebenenfalls anschließend eine Säure oder ein Metallsalz addiert.

Die neuen Imidazolyl-enolether der allgemeinen Formel I weisen starke fungizide Eigenschaften auf. Dabei zeigen überraschenderweise die erfindungsgemäßen Verbindungen eine erheblich höhere Wirkung als die aus dem Stand der Technik bekannten substituierten 3,3-Dimethyl-1-(imidazol-1-yl)-1-phenoxy-2-(R-oxy)-butane, welche chemisch und wirkungsmäßig naheliegende Verbindungen sind. Die erfindungsgemäßen Stoffe stellen somit eine Bereicherung der Technik dar.

Die erfindungsgemäßen Imidazolyl-enolether sind durch die allgemeine Formel I definiert. In dieser Formel steht Ar für gegebenenfalls einfach oder mehrfach, gleich oder verschieden substituiertes Phenyl und Naphthyl, wobei als Substituenten in Frage kommen : Halogen ; Alkyl mit insbesonders 1 bis 4 Kohlenstoffatomen ; Alkoxy mit insbesondere 1 bis 2 Kohlenstoffatomen ; Halogenalkyl mit insbesondere bis zu 2 Kohlenstoff- und bis zu drei gleichen oder verschiedenen Halogenatomen, wobei als Halogene insbesondere Fluor oder Chlor stehen ; Nitro ; Cyano ; sowie gegebenenfalls durch Halogen, insbesondere Chlor substituiertes Phenyl. R steht für geradkettiges oder verzweigtes Alkyl mit 1 bis 4 Kohlenstoffatomen und X steht für Sauerstoff und die Methylengruppe.

Besonders bevorzugt sind diejenigen Imidazolyl-enolether der allgemeinen Formel I, in denen Ar für Phenyl steht, das gegebenenfalls einfach oder zweifach, gleich oder verschieden substituiert ist durch Fluor, Chlor, Brom, Jod, Methyl, Ethyl, Isopropyl, Trifluormethyl, Nitro, Phenyl oder Chlorphenyl ; R für Methyl, Ethyl, Isopropyl, Isobutyl oder tert.-Butyl steht ; und X die in der Erfindungsdefinition angegebene Bedeutung hat.

Im einzelnen seien außer bei den Herstellungsbeispielen genannten Verbindungen die folgenden

Verbindungen der allgemeinen Formel I genannt :

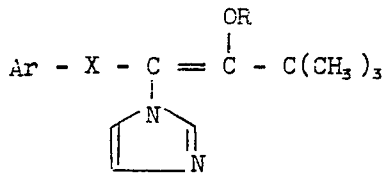

(I)

| Ar | X | R |
|---|---|---|
| 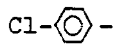 | O | $CH_3$. |
| 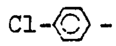 | O | $i-C_3H_7$ |
| 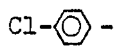 | O | $i-C_4H_9$ |
| 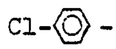 | $CH_2$ | $CH_3$ |
| 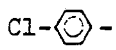 | $CH_2$ | $C_2H_5$ |
| 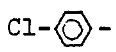 | $CH_2$ | $i-C_3H_7$ |
| 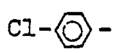 | $CH_2$ | $i-C_4H_9$ |
| 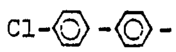 | $CH_2$ | $CH_3$ |
| 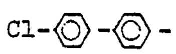 | $CH_2$ | $C_2H_5$ |
| 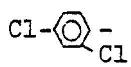 | O | $CH_3$ |
| 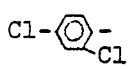 | O | $C_2H_5$ |
| 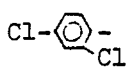 | $CH_2$ | $CH_3$ |
| 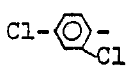 | $CH_2$ | $C_2H_5$ |
| 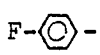 | O | $CH_3$ |
| 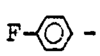 | O | $C_2H_5$ |
| 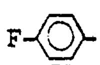 | $CH_2$ | $CH_3$ |
| 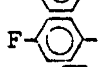 | $CH_2$ | $C_2H_5$ |
| 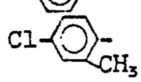 | O | $CH_3$ |
| 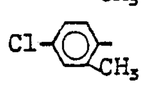 | O | $C_2H_5$ |
| 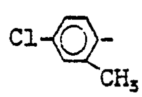 | $CH_2$ | $CH_3$ |
| 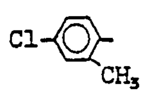 | $CH_2$ | $C_2H_5$ |
| 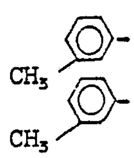 | O | $CH_3$ |
|  | O | $C_2H_5$ |

| Ar | X | R |
|---|---|---|
| 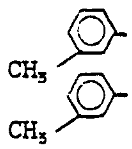 | $CH_2$ | $CH_3$ |
|  | $CH_2$ | $C_2H_5$ |
| 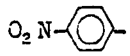 | O | $CH_3$ |
| 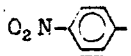 | O | $C_2H_5$ |
| 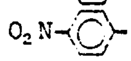 | $CH_2$ | $CH_3$ |
| 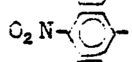 | $CH_2$ | $C_2H_5$ |
| 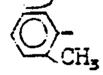 | O | $CH_3$ |
| 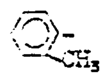 | O | $C_2H_5$ |
| 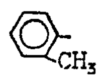 | $CH_2$ | $CH_3$ |
| 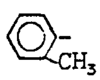 | $CH_2$ | $C_2H_5$ |
| 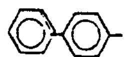 | O | $CH_3$ |
| 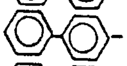 | O | $C_2H_5$ |
| 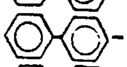 | $CH_2$ | $CH_3$ |
| 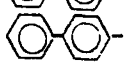 | $CH_2$ | $C_2H_5$ |

Verwendet man beispielsweise 1-(4-Chlorphenoxy)-3,3-dimethyl-1-(imidazol-1-yl)-butan-2-on und Dimethylsulfat als Ausgangsstoffe, so kann der Reaktionsablauf durch das folgende Formelschema wiedergegeben werden :

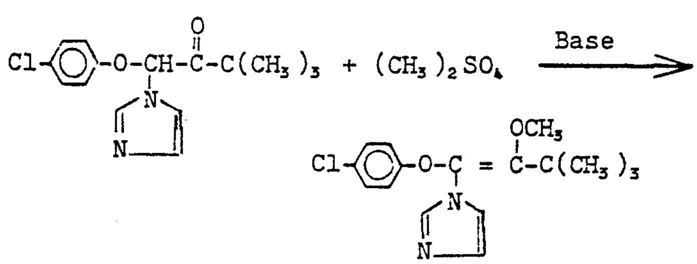


Die bei der Durchführung des erfindungsgemäßen Verfahrens als Ausgangsstoffe zu verwendenden Imidazolyl-ketone sind durch die allgemeine Formel II definiert. In dieser Formel stehen Ar und X vorzugsweise für diejenigen Reste, die bereits im Zusammenhang mit der Beschreibung der erfindungsgemäßen Stoffe der allgemeinen Formel I vorzugsweise für diese Substituenten genannt wurden.

Die Imidazolyl-ketone der allgemeinen Formel II sind bekannt (vergleiche DE-AS 21 05 490 und DE-OS 26 38 470) und können nach den dort angegebenen Verfahren erhalten werden, indem man z.B. entsprechende Halogenketone mit Imidazol in Gegenwart eines Verdünnungsmittels und in Gegenwart eines Säurebinders umsetzt.

Die außerdem für das erfindungsgemäße Verfahren als Ausgangsstoffe benötigten Alkylsulfate bzw. -halogenide sind allgemein bekannte Verbindungen der organischen Chemie. Als Beispiele seien

genannt : Dimethylsulfat, Diethylsulfat, Methylbromid, Methyljodid, Ethylbromid, Ethyljodid, Isopropyljodid und Isobutyljodid.

Für die erfindungsgemäße Umsetzung kommen als Verdünnungsmittel inerte organische Lösungsmittel infrage. Hierzu gehören vorzugsweise aromatische Kohlenwasserstoffe, wie Benzol, Toluol oder Xylol ; halogenierte Kohlenwasserstoffe, wie Methylenchlorid, Tetrachlorkohlenstoff, Chloroform oder Chlorbenzol ; Ester, wie Essigester ; Formamide, wie Dimethylformamid ; sowie Dimethylsulfoxid.

Die erfindungsgemäße Umsetzung wird in Gegenwart einer Base durchgeführt. Hierbei können alle üblichen organischen und insbesondere anorganischen Basen eingesetzt werden, wie vorzugsweise Alkalihydroxide oder Alkalicarbonate, beispielsweise seien Natrium- und Kaliumhydroxid genannt.

Die Reaktionstemperaturen können bei der Durchführung des erfindungsgemäßen Verfahrens in einem größeren Bereich variiert werden. Im allgemeinen arbeitet man zwischen 0 und 100 °C, vorzugsweise zwischen 20 und 80 °C.

Bei der Durchführung des erfindungsgemäßen Verfahrens setzt man vorzugsweise auf 1 Mol Imidazolyl-keton der allgemeinen Formel II 1 bis 2 Mol Alkylsulfat bzw. -halogenid ein. Dabei fallen die Verbindungen der allgemeinen Formel I in Form der geometrischen Isomerengemische an. Die Isolierung der einzelnen geometrischen Isomeren erfolgt nach üblichen Methoden, wie z.B. aufgrund unterschiedlicher Löslichkeit, durch Salzbildung, durch Craig-Verteilung oder durch chromatographische Trennverfahren bzw. durch Kombination dieser Methoden. Eine eindeutige Strukturzuordnung erfolgt aufgrund der $^1$H-NMR-Daten, insbesondere unter Verwendung von Verschiebungsreagenzien.

In einer bevorzugten Ausführungsform wird die erfindungsgemäße Umsetzung in einem Zweiphasensystem, wie beispielsweise wässrige Natron- oder Kalilauge/Toluol oder Methylenchlorid, gegebenenfalls unter Zusatz von 0,1-1 Mol eines Phasen-Transfer-Katalysators, wie beispielsweise Ammonium- oder Phosphoniumverbindungen, beispielsweise seien Benzyl-dodecyl-dimethylammoniumchlorid und Triethyl-benzyl-ammonium-chlorid genannt, durchgeführt (vergleiche auch die Herstellungsbeispiele).

Die erfindungsgemäß herstellbaren Verbindungen der allgemeinen Formel I können in Säureadditions-Salze bzw. Metallsalz-Komplexe überführt werden.

Zur Herstellung von physiologisch verträglichen Säureadditionssalzen der Verbindungen der allgemeinen Formel I kommen vorzugsweise folgende Säuren infrage : Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Michsäure, sowie Sulfonsäuren, wie z.B. p-Toluolsulfonsäure und 1,5-Naphthalindisulfonsäure. Die Säureadditions-Salze der Verbindungen der allgemeinen Formel I können in einfacher Weise nach üblichen Salzbildungsmethoden, z.B. durch Lösen einer Verbindung der allgemeinen Formel I in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel I kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten : Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der allgemeinen Formel I können in einfacher Weise nach üblichen Verfahren erhalten werden, so z.B. durch Lösen des Metallsalzes in Alkohol, z.B. Ethanol und Hinzufügen zur Verbindung der allgemeinen Formel I. Man kann Metallsalz-Komplexe in bekannter Weise, z.B. durch Abfiltrieren, isolieren und gegebenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Getreidemehltau und Getreiderost ; von Uromycets-Arten, wie den Erreger des Bohnenrostes (Uromyces phaseoli) ; sowie von Erysiphe-Arten, wie den Erreger des Gurkenmehltaus (Erysiphe cichoracearum), eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe

mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glykol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

In den nachfolgenden Beispielen werden die nachstehend angegebenen Verbindungen als Vergleichssubstanzen eingesetzt :

(A) 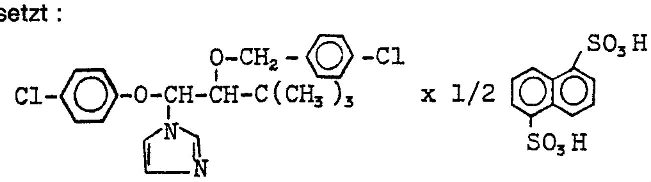

x 1/2

(B) 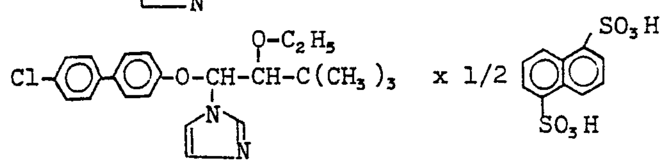

x 1/2

(C) 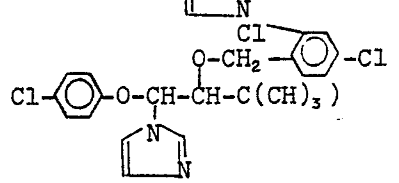 x HCl

(D) 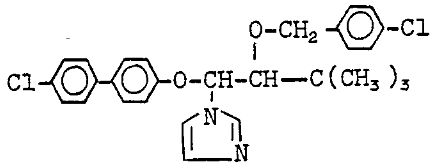 x 1/2 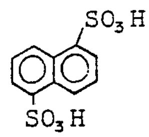

(E) 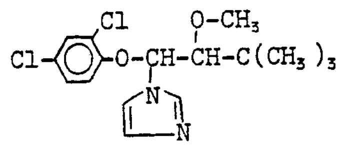

(F) 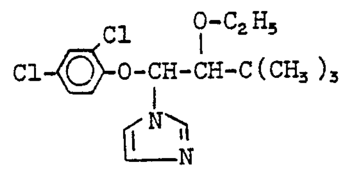

## Beispiel A

Sproßbehandlungs-Test/Getreidemehltau/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkylarylpolyglykolether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21-22 °C und einer Luftfeuchtigkeit von 80-90 % wertet man den Besatz der Pflanzen mit Mehltaupusteln aus. Der Befallsgrad wird in Prozent des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Mehltaubefall ist.

Die Testergebnisse sind der nachfolgenden Tabelle zu entnehmen :

Tabelle A

Sproßbehandlungs-Test/Getreidemehltau/protektiv

| Wirkstoffe | Wirkstoff-konzentration in der Spritz-brühe in Gew.-% | Befall in % der unbe-handelten Kontrolle |
|---|---|---|
| 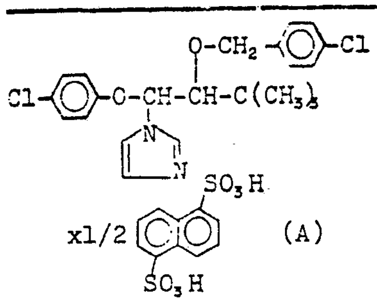 x1/2 (A) (bekannt) | 0,025 | 48,8 |
| 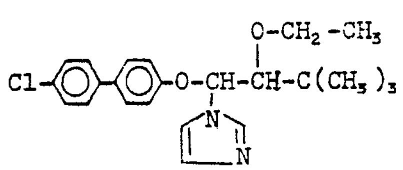 | 0,025 | 60,0 |

x1/2 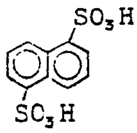 (B)

(bekannt)

| | | |
|---|---|---|
| 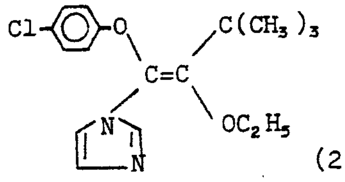 (2) | 0,025 | 0,0 |

Tabelle A

Sproßbehandlungs-Test/Getreidemehltau/protektiv

| Wirkstoffe | Wirkstoff-konzentration in der Spritz-brühe in Gew.-% | Befall in % der unbe-handelten Kontrolle |
|---|---|---|
| 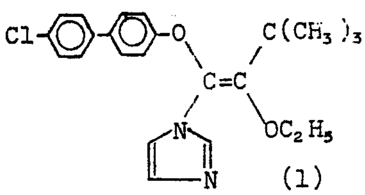 (1) | 0,025 | 23,8 |
| 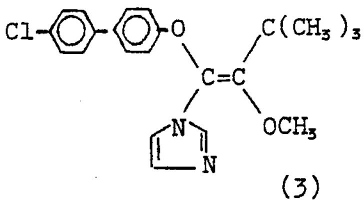 (3) | 0,025 | 0,0 |

Beispiel B

Sproßbehandlungs-Test/Getreiderost/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator Alkylarylpolyglykolether auf und gibt 975 Gewichtteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1 %igem Wasseragar. Nach Antrocknen der Sporensuspension besprüht man die Weizenpflanzen mit der Wirkstoffzubereitung taufeucht und stellt sie zur Inkubation für 24 Stunden bei etwa 20 °C und einer 100 %igen Luftfeuchtigkeit in ein Gewächshaus.

Nach 10 Tagen Verweilzeit der Pflanzen bei einer Temperatur von 20 °C und einer Luftfeuchtigkeit von 80-90 % wertet man den Besatz der Pflanzen mit Rostpusteln aus. Der Befallsgrad wird in Prozent des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Rostbefall ist.

Die Testergebnisse sind der nachfolgenden Tabelle zu entnehmen :

Tabelle B

Sproßbehandlungs-Test/Getreiderost/protektiv

| Wirkstoffe | Wirkstoffkonzentration in der Spritzbrühe in Gew.-% | Befall in % der unbehandelten Kontrolle |
|---|---|---|
| 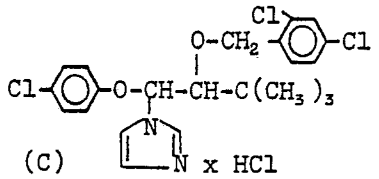 (C) (bekannt) | 0,025 | 100 |
| 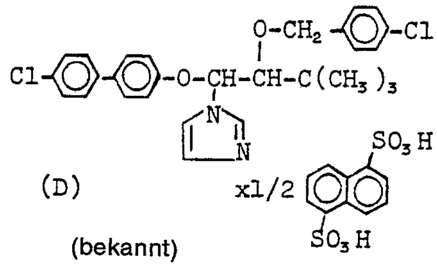 (D) (bekannt) | 0,025 | 65,0 |
| 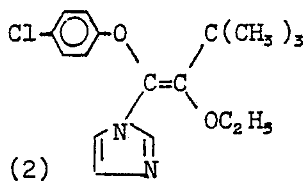 (2) | 0,025 | 37,5 |
| 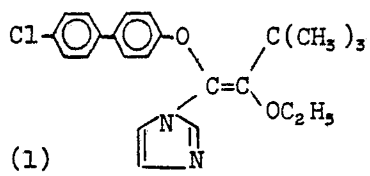 (1) | 0,025 | 0,0 |

Tabelle B (Fortsetzung)

Sproßbehandlungs-Test/Getreiderost/protektiv

| Wirkstoffe | Wirkstoffkonzentration in der Spritzbrühe in Gew.-% | Befall in % der unbehandelten Kontrolle |
|---|---|---|
| 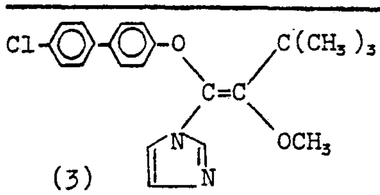 (3) | 0,025 | 14,4 |

## Beispiel C

Erysiphe-Test (Gurken)/protektiv

Lösungsmittel : 4,7 Gewichtsteile Aceton
Emulgator : 0,3 Gewichtsteile Alkylarylpolyglykolether
Wasser : 95,0 Gewichtsteile

Man vermischt die für die gewünschte Wirkstoffkonzentration in der Spritzflüssigkeit nötige

Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden anschließend bei 23 bis 24 °C und bei einer relativen Luftfeuchtigkeit von ca 75 % im Gewächshaus aufgestellt.

Nach 12 Stunden wird der Befall der Gurkenpflanzen bestimmt. Die erhaltenen Boniturwerte werden in Prozent Befall ungerechnet. 0 % bedeutet keinen Befall, 100 % bedeutet, daß die Pflanzen vollständig befallen sind.

Die Testergebnisse sind der nachfolgenden Tabelle zu entnehmen :

Tabelle C

Erysiphe-Test (Gurken)/protektiv

| Wirkstoff | Befall in % bei einer Wirkstoffkonzentration von 0,000 1 % |
|---|---|
| 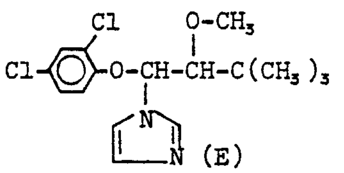 (E) (bekannt) | 37 |
| 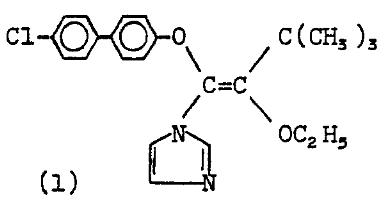 (1) | 29 |

Beispiel D

Uromyces-Test (Bohnenrost)/protektiv

Lösungsmittel : 4,7 Gewichtsteile Aceton
Emulgator : 0,3 Gewichtsteile Alkylarylpolyglykolether
Wasser : 95,0 Gewichtsteile

Man vermischt die für die gewünschte Wirkstoffkonzentration in der Spritzflüssigkeit notwendige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man die jungen Bohnenpflanzen, die sich im 2-Blattstadium befinden, bis zur Tropfnässe. Die Pflanzen verbleiben zum Abtrocknen 24 Stunden bei 20-22 °C und einer relativen Luftfeuchtigkeit von 70 % im Gewächshaus. Anschließend werden sie mit einer wässrigen Uredosporensuspension des Bohnenrosterregers (Uromyces phaseoli) inokuliert und 24 Stunden lang in einer dunklen Feuchtkammer bei 20-22 °C und 100 % relativer Luftfeuchtigkeit inkubiert.

Die Pflanzen werden dann unter intensiver Belichtung für 9 Tage bei 20-22 °C und einer relativen Luftfeuchtigkeit von 70-80 % im Gewächshaus aufgestellt.

10 Tage nach der Inokulation wird der Befall der Pflanzen bestimmt. Die erhaltenen Boniturwerte werden in Prozent Befall umgerechnet. 0 % bedeutet keinen Befall, 100 % bedeutet, daß die Pflanzen vollständig befallen sind.

Die Testergebnisse sind der nachfolgenden Tabelle zu entnehmen :

Tabelle D

Uromyces-Test/protektiv

| Wirkstoff | Befall in % bei einer Wirkstoffkonzentration von : 0,001 % |
|---|---|
| 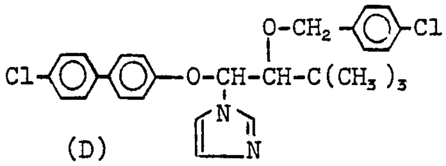 (D) x 1/2 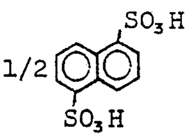 | 100 |
| 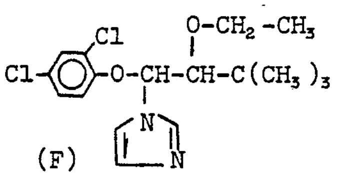 (F) | 75 |
| 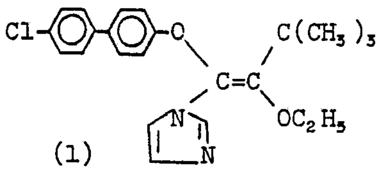 (1) | 9 |
| 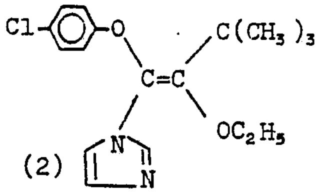 (2) | 62 |

Herstellungsbeispiele

Beispiel 1

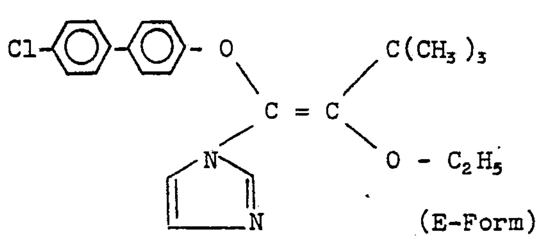

(E-Form)

Zu 36,85 g (0,1 Mol) 1-(4'-Chlor-4-biphenylyloxy)-3,3-dimethyl-1-(imidazol-1-yl)-butan-2-on in 50 ml Dimethylsulfoxid werden 6 g Kaliumhydroxid, in wenig Wasser gelöst, zugetropft. Man läßt kurze Zeit nachrühren und tropft anschließend 16 g (0,11 Mol) Diethylsulfat zu. Dabei wird die Temperatur der Reaktionsmischung auf ca. 40 °C gehalten, danach ca. 1/2 Stunde auf 80 °C. Man läßt abkühlen, versetzt mit Wasser, saugt den kristallinen Niederschlag ab und kristallisiert aus Petrolether um. Man erhält 22 g

(50 % der Theorie) (E)-1-(4'-Chlor-4-biphenylyloxy)-3,3-dimethyl-2-ethoxy-1-(imidazol-1-yl)-1-buten vom Schmelzpunkt 112-113 °C.

In entsprechender Weise können die nachfolgenden Beispiele der allgemeinen Formel I

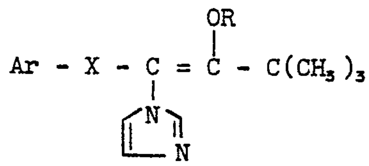

(I)

erhalten werden :

| Bsp.Nr. | Ar | X | R | Schmelz-punkt(°C) |
|---|---|---|---|---|
| 2 | 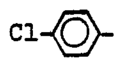 | O | $C_2H_5$ | 94-95 (E-Form) |
| 3 | 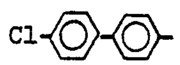 | O | $CH_3$ | 139-42 (E-Form) |

**Ansprüche**

1. Imidazolyl-enolether der allgemeinen Formel I

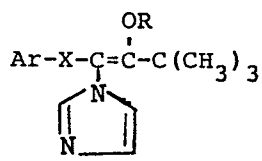

(I)

in welcher

Ar für Phenyl und Naphthyl steht, wobei diese Reste substituiert sein können durch Halogen, Alkyl mit 1 bis 6 und Alkoxy mit 1 bis 4 Kohlenstoffatomen, Halogenalkyl mit 1 bis 4 Kohlenstoffund 1 bis 5 Halogenatomen, ferner durch Nitro, Cyano und schließlich durch gegebenenfalls halogensubstituiertes Phenyl,

R für Alkyl mit 1 bis 4 Kohlenstoffatomen steht und

X für Sauerstoff oder für die Methylengruppe steht, sowie deren Säure- und Metallsalz-Additionsverbindungen.

2. Verfahren zur Herstellung von Imidazolyl-enolethern der allgemeinen Formel I

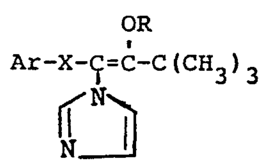

(I)

in welcher

Ar für Phenyl und Naphthyl steht, wobei diese Reste substituiert sein können durch Halogen, Alkyl mit 1 bis 6 und Alkoxy mit 1 bis 4 Kohlenstoffatomen, Halogenalkyl mit 1 bis 4 Kohlenstoffund 1 bis 5 Halogenatomen, ferner durch Nitro, Cyano und schließlich durch gegebenenfalls halogensubstituiertes Phenyl,

R für Alkyl mit 1 bis 4 Kohlenstoffatomen steht und

X für Sauerstoff oder für die Methylengruppe steht, dadurch gekennzeichnet, daß man Imidazolylketone der allgemeinen Formel II

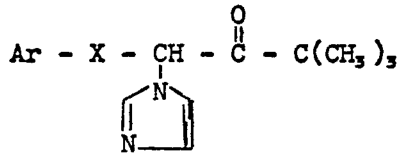

(II)

in welcher

Ar und X die obenangegebene Bedeutung haben, mit entsprechenden Alkylsulfaten bzw. -halogeniden in Gegenwart einer Base und in Gegenwart eines organischen Verdünnungsmittels, oder in einem wäßrigenorganischen Zweiphasensystem in Gegenwart eines Phasentransferkatalysators umsetzt, und gegebenenfalls anschließend eine Säure oder ein Metallsalz addiert.

3. Fungizide Mittel für den Pflanzenschutz, gekennzeichnet, durch einen Gehalt an mindestens einem Imidazolyl-enolether der allgemeinen Formel I in Ansprüchen 1 und 2.

4. Verwendung von Imidazolyl-enolethern der allgemeinen Formel I in Ansprüchen 1 und 2 zur Bekämpfung von Pilzen im Pflanzenschutz.

5. Verfahren zur Herstellung von fungiziden Mitteln für den Pflanzenschutz gemäß Anspruch 3, dadurch gekennzeichnet, daß man Imidazolyl-enolether der allgemeinen Formel I in Ansprüchen 1 und 2 mit Streckmitteln und/oder oberflächenaktiven Mitteln vermischt.

**Claims**

1. Imidazolyl-enol ethers of the general formula I

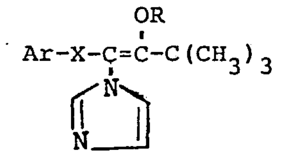

(I)

in which

Ar represents phenyl and naphthyl, it being possible for these radicals to be substituted by halogen, alkyl with 1 to 6 carbon atoms and alkoxy with 1 to 4 carbon atoms, halogenoalkyl with 1 to 4 carbon atoms and 1 to 5 halogen atoms, furthermore by nitro or cyano and finally by optionally halogen-substituted phenyl,

R represents alkyl with 1 to 4 carbon atoms and

X represents oxygen or the methylene group, and acid addition compounds and metal salt addition compounds thereof.

2. Process for the preparation of imidazolyl-enol ethers of the general formula I

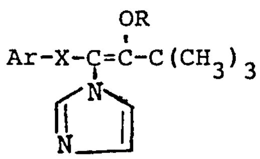

(I)

in which

Ar represents phenyl and naphthyl, it being possible for these radicals to be substituted by halogen, alkyl with 1 to 6 carbon atoms and alkoxy with 1 to 4 carbon atoms, halogenoalkyl with 1 to 4 carbon atoms and 1 to 5 halogen atoms, furthermore by nitro or cyano and finally by optionally halogen-substituted phenyl,

R represents alkyl with 1 to 4 carbon atoms and

X represents oxygen or the methylene group, characterised in that imidazolyl ketones of the general formula II

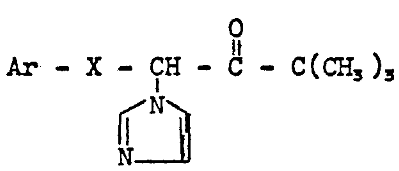

(II)

in which

Ar and X have the meaning indicated above, are reacted with corresponding alkyl sulphates or halides in the presence of a base and in the presence of an organic diluent, or in an aqueous-organic two-phase system in the presence of a phase transfer catalyst, and an acid or a metal salt is then optionally added on.

3. Fungicidal agents for plant protection, characterised in that they contain at least one imidazolylenol ether of the general formula I in Claims 1 and 2.

4. Use of imidazolyl-enol ethers of the general formula I in Claims 1 and 2 for combating fungi in plant protection.

5. Process for the preparation of fungicidal agents for plant protection according to Claims 1 and 2, characterised in that imidazolyl-enol ethers of the general formula I in Claims 1 and 2 are mixed with extenders and/or surface-active agents.

**Revendications**

1. Ether-oxyde d'imidazolyl-énol de formule générale I :

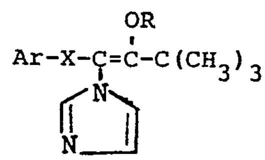

(I)

dans laquelle

Ar représente un groupe phényle et naphtyle, ces radicaux pouvant être substitués par de l'halogène, par un groupe alkyle ayant 1 à 6 et alcoxy ayant 1 à 4 atomes de carbone, halogénoalkyle ayant 1 à 4 atomes de carbone et 1 à 5 atomes d'halogènes, ainsi que par un groupe nitro, cyano et enfin par un groupe phényle éventuellement substitué par de l'halogène,

R représente un groupe alkyle ayant 1 à 4 atomes de carbone, et

X est un atome d'oxygène ou le groupe méthylène, ainsi que leurs composés d'addition d'acides et de sels de métaux.

2. Procédé pour produire des éthers-oxydes d'imidazolyl-énols de formule générale I :

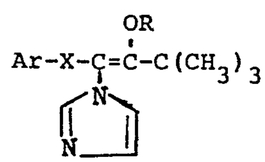

(I)

dans laquelle

Ar représente un groupe phényle et naphtyle, ces radicaux pouvant être substitués par de l'halogène, par un groupe alkyle ayant 1 à 6 et alcoxy ayant 1 à 4 atomes de carbone, halogénoalkyle ayant 1 à 4 atomes de carbone et 1 à 5 atomes d'halogènes, ainsi que par un groupe nitro, cyano et enfin par un groupe phényle éventuellement substitué par de l'halogène,

R représente un groupe alkyle ayant 1 à 4 atomes de carbone, et

X est un atome d'oxygène ou le groupe méthylène, caractérisé en ce qu'on fait réagir des imidazolyl-cétones de formule générale II

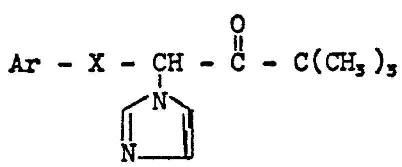

(II)

dans laquelle

Ar et X ont le sens indiqué ci-dessus, avec des sulfates ou halogénures d'alkyles correspondants en présence d'une base et en présence d'un diluant organique, ou dans un système hydro-organique à deux phases en présence d'un catalyseur de transfert des phases, et éventuellement ensuite on fixe par addition un acide ou un sel de métal.

3. Agent fongicide pour la protection des plantes, caractérisé en ce qu'il contient au moins un éther-oxyde d'imidazolylénol de formule générale I selon les revendications 1 et 2.

4. Application des éthers-oxydes d'imidazolyl-énols de formule générale I des revendications 1 à 2 à la lutte contre les champignons pour la protection des plantes.

5. Procédé de production d'agents fongicides pour la protection des plantes selon la revendication 3, caractérisé en ce qu'on mélange des éthers-oxydes d'imidazolyl-énols de formule générale I des revendications 1 et 2 avec des agents d'allongement et/ou des agents tensio-actifs.